(19) Europäisches Patentamt
European Patent Office
Office européen des brevets

(11) Numéro de publication: **0 225 311**
**A2**

(12) # DEMANDE DE BREVET EUROPEEN

(21) Numéro de dépôt: **86870177.2**

(51) Int. Cl.⁴: **C 07 C 125/065**

(22) Date de dépôt: **01.12.86**

(30) Priorité: **02.12.85 FR 8517805**

(43) Date de publication de la demande:
**10.06.87 Bulletin 87/24**

(84) Etats contractants désignés:
**AT BE CH DE ES FR GB GR IT LI LU NL SE**

(71) Demandeur: **SANOFI, société anonyme**
**40, Avenue George V**
**F-75008 Paris (FR)**

(72) Inventeur: **Descamps, Marcel**
**Place des Carmes 8/301**
**B-1300 Wavre (BE)**

**Nisato, Dino**
**4, Impasse de l'Olivette**
**F-34690 Saint Georges d'Orques (FR)**

**Verstraeten, Walter**
**Zwijvegemstraat, 10**
**B-2960 Mechelen (BE)**

(74) Mandataire: **Cauchie, Daniel**
**c/o S.A. LABAZ-SANOFI N.V. Avenue De Béjar, 1**
**B-1120 Bruxelles (BE)**

(54) **Dérivés d'acide amino-4 butanoïque, leur procédé de préparation et leur utilisation.**

(57) L'invention se rapporte à de nouveaux dérivés d'acide (4S)-amino-4 butanoïque de formule générale :

OH

$R_2-CH-CH-CH_2-CO_2R_1$ I

NHR

dans laquelle :
R représente un groupement N-protecteur,
$R_1$ représente l'hydrogène, un atome de métal alcalin tel que lithium, sodium ou potassium ou un groupement labile,
$R_2$ représente un groupement de formule :

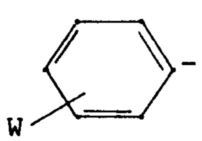

ou

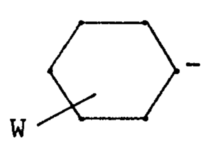

dans laquelle W représente l'hydrogène, le groupe hydroxy ou un radical alkyle ayant de 1 à 4 atomes de carbone ou alcoxy ayant de 1 à 4 atomes de carbone, ainsi qu'à un procédé de préparation desdits dérivés.

Les dérivés d'acide amino-4 butanoïque selon l'invention sont utiles comme intermédiaires de synthèse pour la préparation de peptides dérivés d'analogues de statine.

EP 0 225 311 A2

**Description**

DERIVES D'ACIDE AMINO-4 BUTANOIQUE, LEUR PROCEDE DE PREPARATION ET LEUR UTILISATION.

La présente invention se rapporte, d'une manière générale, à de nouveaux dérivés d'acide (4S)-amino-4 butanoïque de formule générale :

$$R_2-\underset{NHR}{CH}-\overset{OH}{CH}-CH_2-CO_2R_1 \qquad I$$

dans laquelle :
R représente un groupement N-protecteur,
$R_1$ représente l'hydrogène, un atome de métal alcalin tel que lithium, sodium ou potassium ou un groupement labile,
$R_2$ représente un groupement de formule :

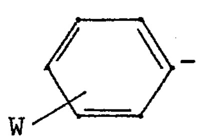

ou

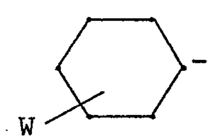

dans laquelle W représente l'hydrogène, le groupe hydroxy ou un radical alkyle ayant de 1 à 4 atomes de carbone ou alcoxy ayant de 1 à 4 atomes de carbone.

Lorsque $R_2$ représente un groupement hydroxy-phényle, le p-hydroxy-phényle constitue un groupement préféré.

Dans le présent contexte les termes repris ci-dessous comportent la signification suivante :
"groupement N-protecteur" désigne un groupement facilement éliminable fixé sur l'azote d'un groupement amino tels que, à titre d'exemple, un groupement formyle, un groupement alkylcarbonyle tels qu'acétyle ou propionyle, un groupement alkoxycarbonyle tel que tertiobutoxycarbonyle, un groupement alkoxyalkylcarbonyle tel que méthoxyacétyle ou méthoxypropionyle, un groupement alkoxycarbonyle substitué tel que trichloro-2,2,2 éthoxycarbonyle, un groupement aralkyloxycarbonyle tel que benzyloxycarbonyle, un groupement aralkyloxycarbonyle substitué tel que p-nitrobenzyloxycarbonyle, un groupement trityle ou méthoxytrityle ou un groupement arylsulfonyle tel que p-toluènesulfonyle, le groupement tertiobutoxycarbonyle (BOC) constituant un groupement préféré,
"groupement labile" désigne un groupement estérifiant facilement éliminable tel qu'un groupement alkyle ayant de 1 à 4 atomes de carbone par exemple méthyle, éthyle, n-propyle, isopropyle, n-butyle, isobutyle ou tertiobutyle ou un groupement aralkyle substitué ou non tel que benzyle ou xylyle.

Les composés de formule I ci-dessus en raison de l'asymétrie des carbones 3 et 4 peuvent se trouver sous la forme de diastéréoisomères (3S,4S) ou (3R,4S).

En conséquence, l'invention se rapporte à la fois aux isomères (3S, 4S) et (3R,4S) des composés de formule I, sous forme séparée ou sous forme de mélanges.

On a décrit dans le brevet U.S. No. 4.485.099 ou la demande de brevet européen No. 114.993 des peptides dérivés d'analogues d'acide (3S,4S)-hydroxy-3 amino-4 méthyl-6 heptanoïque ou statine, ces peptides étant utiles pour leurs potentialités antihypertensives par inhibition de l'enzyme de conversion de la rénine en angiotensine.

La synthèse de ces peptides nécessite une voie d'accès aisée vers des analogues d'acide hydroxy-3 amino-4 méthyl-6 heptanoïque, en particulier vers les isomères (3S,4S) de ces analogues.

On a trouvé, dans le cadre de l'invention, que les dérivés amino-4 butanoïques N-protégés de l'invention constituent des composés particulièrement intéressants pour la préparation de peptides dérivés d'analogues de statine en utilisant un procédé semblable à celui décrit dans le brevet U.S. No. 4.485.099 ou la demande de brevet européen No. 114.993.

En effet, on a pu mettre en évidence que des peptides préparés à partir de composés de l'invention présentent une action inhibitrice de l'activité de la rénine plasmatique humaine les rendant particulièrement utiles pour le traitement de l'hypertension artérielle.

Par conséquent, un autre objet de l'invention se rapporte à l'utilisation des composés de formule I pour la préparation de peptides dérivés d'analogues de statine.

Les dérivés amino-4 butanoïques N-protégés de l'invention peuvent être préparés facilement et avec grande pureté, notamment les isomères (3S,4S), selon un procédé extrapolable sur le plan industriel.

Selon l'invention, on prépare les dérivés d'acide (3S,4S ; 3R,4S)-hydroxy-3 amino-4 butanoïque N-protégés de formule I :

a) lorsque $R_1$ représente un groupement labile, par hydrogénation d'un dérivé (4S)-oxo-3 amino-4

butanoïque protégé de formule générale :

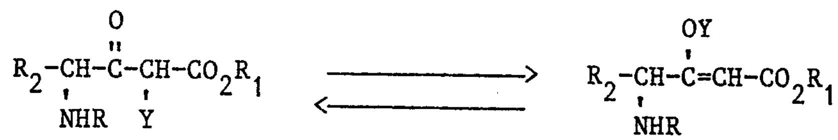

II II'

dans laquelle R et $R_2$ ont la même signification que précédemment, le groupement hydroxy éventuellement présent étant protégé, par exemple par un radical benzyle, $R_1$ représente un groupement labile et Y représente l'hydrogène ou un atome de métal alcalin par exemple lithium, sodium ou potassium et ce, en présence d'un catalyseur par exemple le nickel de Raney, à température ambiante dans un solvant approprié par exemple le méthanol et sous pression par exemple environ 7 bars pour obtenir les composés désirés sous forme de mélange de diastéréoisomères,

b) lorsque $R_1$ représente un atome de métal alcalin, par saponification au moyen d'un hydroxyde de métal alcalin par exemple l'hydroxyde de lithium, sodium ou potassium, d'un ester d'acide (3S,4S ; 3R,4S)-hydroxy-3 amino-4 butanoïque protégé préparé au paragraphe a) précédent et ce dans un solvant approprié tel que le dioxanne aqueux et à température ambiante, pour obtenir les composés désirés sous forme de mélange de diastéréoisomères,

c) lorsque $R_1$ représente hvdrogène par acidification à température ambiante au moyen d'un acide fort tel que l'acide chlorhydrique, d'un sel d'acide (3S,4S ; 3R,4S)-hydroxy-3 amino-4 butanoïque protégé préparé au paragraphe b) précédent, pour obtenir les composés désirés sous forme de mélange de diastéréoisomères,

ces diastéréoisomères pouvant, si on le désire, être séparés par exemple par chromatographie.

Les dérivés (4S)-oxo-3 amino-4 butanoïques protégés de formule II-II′ peuvent être préparés à partir d'un acide aminé protégé de formule générale :

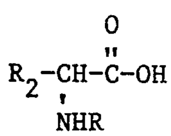

III

dans laquelle R et $R_2$ ont la même signification que dans la formule II-II′, lequel peut être obtenu en protégeant de manière classique les groupements amino et hydroxy de l'acide aminé L correspondant. De tels acides aminés L peuvent être préparés de manière analogue à celle décrite dans le brevet belge No. 845.187.

L'acide aminé protégé de formule III après réaction avec le N,N-thionyldiimidazole, suivant une méthode analogue à celle décrite dans Bull. Soc. Chim. France 1964, pp 945-951, fournit l'imidazolide protégé de formule générale :

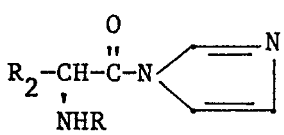

IV

dans laquelle R et $R_2$ ont la même signification que dans la formule II-II′.

On fait ensuite réagir cet imidazolide de formule IV avec un énolate magnésien d'un monoester d'acide malonique de formule générale :

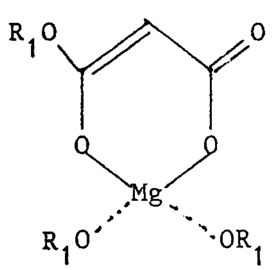

V

dans laquelle $R_1$ a la même signification que précédemment , la réaction ayant lieu à température ambiante et

dans un éther tel que le tétrahydrofuranne éventuellement en présence d'un solvant aprotique polaire tel que le diméthylsulfoxide ou le N,N-diméthylformamide pour donner un complexe que l'on hydrolyse en présence d'un acide fort. par exemple l'acide chlorhydrique, pour ainsi fournir les esters de dérivés d'acide (4S)-oxo-3 amino-4 butanoïque protégés de formule II-II' dans laquelle Y représente hydrogène.

Un tel mélange éther/solvant aprotique polaire est généralement indiqué pour la préparation des esters de formule II-II' en question.

Dans ce cas, on utilise de préférence un mélange tétrahydrofuranne/diméthylsulfoxide qui permet l'obtention de rendements importants du produit désiré sous forme pure alors que dans le tétrahydrofuranne seul. les rendements sont généralement plus faibles.

On forme alors le sel de métal alcalin des esters, de formule II-II', dans laquelle Y représente un atome de métal alcalin, par réaction des dérivés butanoates de formule II-II' dans laquelle Y représente hydrogène, la réaction ayant lieu en milieu aqueux contenant éventuellement un solvant organique tel que l'hexane avec un hydroxyde de métal alcalin. par exemple l'hydroxyde de lithium, de sodium ou de potassium et à température inférieure à 20° C, puis on sépare le sel formé du milieu réactionnel.

Les sels de métaux alcalins ainsi obtenus peuvent être utilisés notamment pour la régénération des esters de formule II-II' dans laquelle Y représente hydrogène et ce, par acidification de leur solution aqueuse au moyen d'un acide fort, par exemple l'acide chlorhydrique.

Dans cette application, l'isolation intermédiaire des sels de métaux alcalins en question constitue une méthode de choix pour la purification des esters de formule II-II' dans laquelle Y représente hydrogène, obtenus à l'état brut lors de la mise en oeuvre du procédé de préparation décrit précédemment.

Cette purification par passage au sel de métal alcalin correspondant permet d'obtenir, avec des rendements importants, les esters de formule II-II' dans laquelle Y représente hydrogène.

Les esters de formule II-II' dans laquelle Y représente hydrogène ainsi purifiés pourront donc être réduits ultérieurement par l'hydrogène catalytique pour donner les esters correspondants de formule I sous forme de mélange de diastéréoisomères.

Il est possible, à ce stade, d'effectuer la séparation des deux diastéréoisomères par exemple par chromatographie.

En outre, on a constaté que la préparation des esters de formule I peut être effectuée directement à partir des sels de formule II-II' par réduction catalytique de ces composés par l'hydrogène. De cette manière, on évite une étape de procédé à savoir la régénération des esters de formule II-II', dans laquelle Y représente hydrogène après purification par passage intermédiaire au sel de métal alcalin.

Les composés de formule I peuvent être utilisés pour la préparation de peptides selon les méthodes habituelles de la chimie des peptides.

En particulier, ces peptides peuvent être obtenus selon un procédé étape par étape à partir du C terminal. Le produit de départ est avantageusement un composé de formule I sous forme d'ester sur lequel on condense l'aminoacide suivant de la séquence. Après libération de la fonction amine du dipeptide, on procède à l'élongation de la chaîne peptidique par couplage de l'acide aminé suivant, convenablement protégé. Chaque phase de couplage est suivie d'une opération sélective de libération de l'amine qui va entrer en réaction lors de la création de la liaison peptidique suivante. Les différentes opérations de couplage sont effectuées soit en utilisant un ester activé de l'aminoacide à coupler, soit en utilisant l'aminoacide N-protégé en présence de dicyclohexylcarbodiimide.

Les phases de déprotection sélective de l'amine sont effectuées selon la nature du groupe protecteur utilisé soit par hydrogénolyse, soit par acidolyse en milieu acide fort tel que l'acide trifluoroacétique. Lorsque l'acide aminé à introduire dans la séquence possède dans sa chaîne latérale une fonction susceptible de réagir, il convient de bloquer celle-ci par un groupe protecteur convenable que l'on élimine ultérieurement.

Enfin, le peptide sous forme acide peut être obtenu par saponification des esters correspondants en milieu alcalin dilué.

Les exemples non limitatifs suivants illustrent la préparation des composés de l'invention ainsi que leur utilisation.

<u>Acides aminés ou groupes protecteurs ou activateurs.</u>

a) Acides aminés
Ala Alanine
Nle Norleucine
Phe Phenylalanine
Sta Statine configuration (3S,4S)

b) Groupes protecteurs ou activateurs
APHBA Acide (3S,4S)-hydroxy-3 amino-4 phényl-4 butanoique
ONSU Ester N-hydroxysuccinimidique
BOC Tertiobutoxycarbonyle
ONP Ester p-nitrophényle

On a également utilisé les abréviations suivantes :

BOP Benzotriazolyloxy-tris-diméthylaminophosphonium hexafluorophosphate

C.C.M. Chromatographie sur couche mince
C.L.H.P. Chromatographie liquide haute pression
HOBt p-Hydroxybenzotriazole
P.F. Point de Fusion
NEM N-ethylmorpholine
R.M.N. Résonance magnétique nucléaire

PREPARATIONS

A)N,N'-thionyldiimidazole

Dans un ballon d'un litre muni d'un agitateur et d'une ampoule à décanter fermée par une garde desséchante, on place 13,6g (0,2 mole) d'imidazole dissous dans 150ml de tétrahydrofuranne.

On ajoute alors, sous agitation, une solution de 6g de chlorure de thionyle dans 50ml de tétrahydrofuranne. La précipitation du chlorhydrate d'imidazole commence immédiatement.

Après 20 minutes d'agitation, on filtre et on lave le précipité avec 50ml de tétrahydrofuranne. On obtient ainsi une solution limpide de N,N'-thionyldiimidazole utilisée telle quelle pour l'opération suivante.

B) Imidazolide de l'acide N-BOC-amino-2 phényl-2 acétique sous forme L.

Dans un ballon d'un litre muni d'un agitateur et d'une ampoule à décanter, on place la solution obtenue précédemment puis on ajoute, goutte à goutte, une solution de 12,5g (0,05 mole) d'acide N-BOC-amino-2 phényl-2 acétique sous forme L dans 20ml de tétrahydrofuranne.

On poursuit l'agitation durant 20 min. en évacuant par succion sous vide (environ 2660 Pa) l'anhydride sulfureux formé. On obtient ainsi une solution légèrement trouble d'imidazolide de l'acide N-BOC-amino-2 phényl-2 acétique sous forme L utilisé brut.

C) Malonate acide de méthyle.

Dans un ballon de 4 litres, muni d'un agitateur et d'une ampoule à décanter, on place 660g (5 moles) de malonate de diméthyle. On ajoute alors en 8 heures environ, une solution à 20°C de 281g d'hydroxyde de potassium dans 2 litres de méthanol.

Après 15 à 16 heures d'agitation à la température ambiante, on filtre le précipité de sel potassique du malonate acide de méthyle et on le lave soigneusement à l'éther éthylique.

On reprend le précipité par 750ml d'eau et on acidifie avec de l'acide chlorhydrique dilué jusqu'à pH = 2 à 3 tout en refroidisant par un mélange glace/méthanol. On extrait 3 fois avec de l'éther éthylique, sèche la phase éthérée et évapore à sec.

On isole ainsi 156g de malonate acide de méthyle brut, ce qui représente un rendement de 26%.

Après une distillation sous $5.10^{-2}$ Torr à 80-85°C, on isole 135g de produit pur ce qui représente un rendement de 23%.

$n_D^{23} = 1,4300$

R.M.N. : conforme

Dosage protométrique : 97,17%

D) Enolate magnésien du malonate acide de méthyle.

Dans un ballon de 2 litres, muni d'un agitateur, d'un réfrigérant et d'une ampoule à décanter, on introduit successivement 4,8g (0,2 mole) de magnésium, 0,2ml de tétrachlorure de carbone et 10ml de méthanol.

A ce mélange, on ajoute, sous agitation, 10ml d'une solution de 23,5g (0,2 mole) de malonate acide de méthyle dans 50ml de méthanol.

La réaction démarre spontanément. Lorsqu'elle se calme, on ajoute le reste de la solution de malonate de façon à tenir un léger reflux. Après introduction, on chauffe au bain-marie durant 8 heures, on ajoute 200ml de tétrahydrofuranne et on poursuit le chauffage au bain-marie durant 12 heures. On distille alors les solvants, d'abord à pression atmosphérique ensuite sous 2660 Pa environ. Arrivé à sec, on ajoute 100ml de benzène et on distille à pression atmosphérique puis sous vide. Finalement, on ajoute 100ml de tétrahydrofuranne.

On obtient ainsi une suspension d'énolate magnésien du malonate acide de méthyle.

EXEMPLE 1

Préparation des (3S,4S)- et (3R,4S)-hydroxy-3 N-BOC-amino-4 phényl-4 butanoates deméthyle.

I. Sel sodique de (4S)-oxo-3 N-BOC-amino-4 phényl-4 butanoate de méthyle.

On prépare l'imidazolide de l'acide N-BOC-amino-2 phényl-2 acétique sous forme L à partir de 13,6g d'imidazole et de 12,5g d'acide N-BOC-amino-2 phényl-2 acétique sous forme L suivant la méthode décrite au paragraphe B précédent. De même, on prépare l'énolate magnésien du malonate acide de méthyle à partir de 24.7g de malonate acide de méthyle selon la méthode décrite au paragraphe D précédent.

On ajoute la solution d'imidazolide à la suspension d'énolate magnésien dans le tétrahydrofuranne puis on ajoute 130ml de diméthylsulfoxyde. Le mélange se clarifie et tout se dissout.

Après 4 heures d'agitation à température ambiante, on acidifie au moyen d'acide chlorhydrique 1N jsuqu'à

pH neutre. On agite 30 minutes à température ambiante afin de parfaire l'hydrolyse.

On décante et on extrait 3 fois à l'éther éthylique. On lave la phase éthérée successivement avec de l'eau, de l'eau bicarbonatée puis avec de l'eau. Après sèchage sur sulfate de sodium, on évapore à sec et on obtient 12,2g de (4S)-oxo-3 N-BOC-amino-4 phényl-4 butanoate de méthyle brut.

On reprend le dérivé de butanoate de méthyle brut dans 60ml d'hexane et 20ml d'eau. On agite le mélange hétérogène et à température inférieure à 20°C, on ajoute 10ml d'une solution d'hydroxyde de sodium à 30%. La précipitation du sel sodique commence après environ 5 minutes. Afin de compléter la précipitation, on continue l'agitation durant 15 minutes.

On filtre le précipité et on le lave avec de l'eau glacée et de l'éther éthylique. Après sèchage, on obtient le sel sodique de (4S)-oxo-3 N-BOC-amino-4 phényl-4 butanoate de méthyle, légèrement soluble dans l'éther ethylique.

P.F. : 182-184°C

II) (3S,4S)- et (3R,4S)-hydroxy-3 N-BOC-amino-4 phényl-4 butanoate de méthyle.

On dissout 6g de sel sodique de (4S)-oxo-3 N-BOC-amino-4 phényl-4 butanoate de méthyle dans 150ml de méthanol. Après addition de 1g de nickel de Raney, on hydrogène sous une pression de 7 bars durant 24 heures.

On neutralise l'alcalinité par de l'acide acétique et on filtre. Après évaporation, on reprend par l'hexane afin d'éliminer les sels minéraux puis on évapore l'hexane.

On obtient ainsi un mélange de deux diastéréosiomères (3S,4S ; 3R, 4S) d'hydroxy-3 N-BOC-amino-4 phényl-4 butanoate de méthyle brut.

On sépare alors ce mélange brut par chromatographie sur colonne de silice (diamètre : 90mm, hauteur : 500mm) en utilisant un mélange acétate d'éthyle/hexane : 10:90.

Après évaporation des différentes fractions, on isole les deux diastéréoisomères qui cristallisent au repos. On obtient ainsi :

a) le (3S,4S)-hydroxy-3 N-BOC-amino-4 phényl-4 butanoate de méthyle

P.F. : 94-95°C (éther diisopropylique)

$\alpha_D^{25}$ = + 7,9° (C=1, méthanol)

b) le (3R,4S)-hydroxy-3 N-BOC-amino-4 phényl-4 butanoate de méthyle

P.F. : 94-95°C (éther diisopropylique)

$\alpha_D^{25}$ = + 1,8° (C=1, méthanol)

EXEMPLE 2

Préparation des (3S,4S)- et (3R,4S)-hydroxy-3 N-BOC-amino-4 phényl-4 butanoate de méthyle.

I. (4S)-oxo-3 N-BOC-amino-4 phényl-4 butanoate de méthyle

On prépare l'imidazolide de l'acide N-BOC-amino-2 phényl-2 acétique, forme L à partir de 136g d'imidazole et 125g d'acide N-BOC-amino-2 phényl-2 acétique, forme L suivant la méthode décrite au paragraphe B précédent. De même, on prépare l'énolate magnésien du malonate acide de méthyle à partir de 236g de malonate acide de méthyle selon la méthode décrite au paragraphe D précédent.

On ajoute la solution d'imidazolide à la suspension d'énolate magnésien dans le tétrahydrofuranne puis on ajoute 1,3 litre de diméthylsulfoxyde. Le mélange de clarifie et tout se dissout.

Après 4 heures d'agitation à température ambiante, on acidifie au moyen d'acide chlorhydrique 1N jusqu'à pH neutre. On agite durant 30 minutes à température ambiante afin de parfaire l'hydrolyse. On décante et on extrait 3 fois à l'éther éthylique. On lave la phase éthérée successivement avec de l'eau, de l'eau bicarbonatée puis avec de l'eau. Après sèchage sur sulfate de sodium, on évapore à sec et on obtient le (4S)-oxo-3 N-BOC-amino-4 phényl-4 butanoate de méthyle brut.

On reprend le dérivé de butanoate de méthyle brut dans 200ml d'éther isopropylique, 200ml d'hexane et 100ml d'eau.

A température inférieure à 20°C, on ajoute sous agitation, 50ml d'hydroxyde de sodium à 30%. Après 15 minutes d'agitation, on filtre le précipité de sel sodique de (4S)-oxo-3 N-BOC-amino-4 phényl-4 butanoate de méthyle formé et on le lave avec de l'hexane. On reprend le précipité mouillé avec un mélange d'eau/hexane et on ajoute de l'acide chlorhydrique 1N jusqu'à pH = 2 à 3.

On extrait 2 fois à l'hexane et on lave la phase organique avec une solution de bicarbonate de sodium à 10%. Après sèchage et évaporation, on isole 80g de (4S)-oxo-3 N-BOC-amino-4 phényl-4 butanoate de méthyle sous forme huileuse.

Rendement : 52% calculé au départ de l'acide N-BOC-amino-2 phényl-2 acétique, forme L.

$\alpha_D^{25}$ = + 77° (C=1, méthanol)

P.F. : 97-98°C

R.M.N. et C.C.M. : conformes

II) (3S,4S)- et (3R,4S)-hydroxy-3 N-BOC-amino-4 phényl-4 butanoate de méthyle.

On dissout dans 400ml de méthanol anhydre 80g de (4S)-oxo-3 N-BOC-amino-4 phényl-4 butanoate de méthyle pur obtenu à partir de son sel sodique. Après addition d'environ 6g de nickel de Raney, on hydrogène

durant 72 heures sous une pression d'environ 7 bars et à température d'environ 20°C.

Après cette opération, on filtre et on évapore à sec pour obtenir un mélange de deux diastéréoisomères (3S,4S ; 3R,4S) d'hydroxy-3 N-BOC-amino-4 phényl-4 butanoate de méthyle sous forme huileuse.

On sépare alors cette huile par chromatographie sur colonne de gel de silice (diamètre : 90mm, hauteur : 500mm) en utilisant un mélange acétate d'éthyle/hexane 10:90.

Après évaporation des différentes fractions on isole les deux diastéréoisomères qui cristallisent au repos. On obtient ainsi :

a) le (3S,4S)-hydroxy-3 N-BOC-amino-4 phényl-4 butanoate de méthyle

Rendement : 12% calculé à partir du dérivé oxo-3

P.F. : 94-95° C (éther diisopropylique)

$\alpha_D^{25}$ = + 7,9° (C = 1, méthanol)

b) le (3R,4S)-hydroxy-3 N-BOC-amino-4 phényl-4 butanoate de méthyle

Rendement : 45% calculé à partir du dérivé oxo-3

P.F. : 94-95° C (éther diisopropylique)

$\alpha_D^{25}$ = + 1,8° (C = 1, méthanol)

L'Exemple suivant illustre la préparation d'un peptide à partir d'un composé de formule I.

EXEMPLE I

Préparation du BOC-Phe-Nle-APHBA-Ala-Sta-$OCH_3$ (SR 44205)

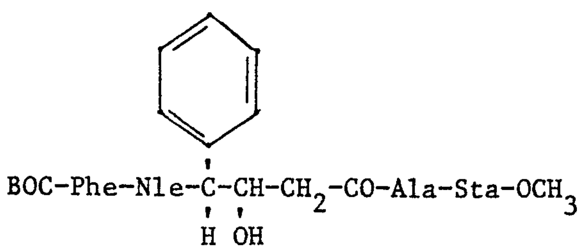

a) (N-BOC-Norleucylamino)-4 hydroxy-3 phényl-4 butanoate de méthyle

On dissout à 0°C 1g (0,0032 mole) de (3S,4S)-hydroxy-3 (N-BOC-amino)-4 phényl-4 butanoate de méthyle, en partie racémisé, dans 10ml d'acide trifluoroacétique pur. Après 15 minutes à 0°C, on évapore rapidement l'acide sous pression réduite , sans chauffer jusqu'à ce que toute vapeur ait disparu. On met alors en suspension dans du chlorure de méthylène refroidi à 0°C le sel ainsi obtenu et on ajoute successivement 1,06g (0,0032 mole) de BOC-Nle-ONSU, 0,620g (0,004 mole) de HOBt puis suffisamment de NEM pour amener le pH de la solution vers 6-7. On réchauffe alors le milieu réactionnel à température ambiante en 2h en contrôlant son pH puis on agite durant 12 à 15 heures. On évapore le solvant à température ambiante au rotavapor de Büchi et on extrait le peptide au moyen d'acétate d'éthyle. On lave alors la phase organique successivement 4 fois avec une solution aqueuse diluée de carbonate de sodium, 2 fois avec de l'eau, 4 fois avec une solution aqueuse diluée de sulfate acide de potassium ensuite 2 fois avec de l'eau et 1 seule fois avec une solution aqueuse de chlorure de sodium. On sèche sur sulfate de magnésium et on purifie par chromatographie sur colonne de gel de silice en éluant par un mélange 1/1 de chlorure de méthylène/acétate d'éthyle. Après trituration dans un mélange éther éthylique/hexane, on obtient 0,629g de (N-BOC-norleucylamino)-4 hydroxy-3 phényl-4 butanoate de méthyle.

b) (N-BOC-Phenylalanyl-norleucylamino)-4 hydroxy-3 phényl-4 butanoate de méthyle.

Dans 5ml d'acide trifluoroacétique on triture à 0°C 0,453g ($1,07 \times 10^{-3}$ mole) de (N-BOC-norleucylamino)-4 hydroxy-3 phényl-4 butanoate de méthyle obtenu précédemment. Après 15 minutes de contact, on évapore l'acide en excès à température ambiante au moyen d'un rotavapor de Büchi. On dissout le sel ainsi obtenu dans 10ml de chlorure de méthylène à 0°C et on ajoute successivement 0,498g (1,2 équivalent) de BOC-Phe-ONP et 0,197g (1,2 équivalent) de HOBt.

On neutralise le milieu réactionnel avec de la N-méthylmorpholine et on laisse revenir à température ambiante tout en ajustant le pH à environ 7 avec une quantité supplémentaire de N-méthylmorpholine.

On évapore à sec puis on ajoute une solution aqueuse de carbonate de sodium. Après extraction avec 3 volumes d'acétate d'éthyle, on lave l'extrait avec une solution aqueuse de carbonate de sodium, avec de l'eau puis avec de l'eau contenant du chlorure de sodium. Après séchage sur sulfate de magnésium et évaporation du solvant sous vide, on chromatographie le résidu sur colonne de gel de silice et on élue avec un mélange 50/50 v/v de chlorure de méthylène/acétate d'éthyle. Après trituration dans l'hexane, on obtient 0,324g de (N-BOC-phénylalanyl-norleucylamino)-4 hydroxy-3 phényl-4 butanoate de méthyle sous la forme d'un mélange 80/20 de deux isomères détectés par C.L.H.P.

c) BOC-Phe-Nle-APHBA-Ala-Sta-$OCH_3$

Au moyen d'un mélange méthanol/eau/hydroxyde de sodium, on hydrolyse 0,260g de (N-BOC-phénylalanyl-norleucylamino)-4 hydroxy-3 phényl-4 butanoate de méthyle obtenue précédemment. Après cette opération, on mélange dans le chlorure de méthylène de l'acide trifluoroacétique, Ala-Sta-$OCH_3$ (préparé à partir de 0,204g ou 1,2 équivalent de BOC-Ala-Sta-$OCH_3$), 0,210g de BOP et l'acide obtenu ci-dessus. On neutralise le milieu avec de la diisopropyléthylamine et on agite à température ambiante pendant 15h. Après évaporation du solvant et extraction avec de l'acétate d'éthyle, on concentre et chromatographie deux fois le résidu sur colonne de gel de silice en utilisant un mélange 95/5 v/v d'acétate d'éthyle/méthanol comme éluant.

On collecte et évapore les fractions qui apparaissent homogènes en C.C.M. et on triture le résidu dans l'éther éthylique.

De cette manière, on obtient le BOC-Phe-Nle-APHBA-Ala-Sta-$OCH_3$.

Rendement : 91% (déterminé par C.L.H.P.)

P.F. : 214-216°C

On peut recristalliser les fractions impures trois fois dans l'acétonitrile chaud.

Rendement : 100% (déterminé par C.L.H.P.)

P.F. : 214-216°C

On a étudié le peptide ainsi obtenu en vue d'en déterminer l'action inhibitrice de l'activité de la rénine plasmatique humaine en utilisant la méthode décrite dans la demande de brevet européen No. 104.964.

Selon ce test, le SR 44205 présente une $CI_{50}$ égale à $10^{-6}$M à pH = 6, $CI_{50}$ représentant la dose de composé à étudier provoquant une inhibition de 50% de l'activité de la rénine plasmatique humaine prise comme référence.

## Revendications

1. Dérivés d'acide (4S)-amino-4 butanoïque de formule générale :

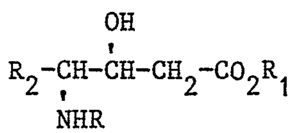

I

dans laquelle :

R représente un groupement N-protecteur,

$R_1$ représente l'hydrogène, un atome de métal alcalin ou un groupement labile,

$R_2$ représente un groupement de formule :

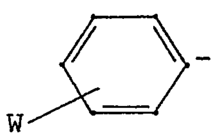

ou

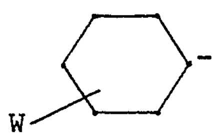

dans laquelle W représente l'hydrogène, le groupe hydroxy ou un radical alkyle ayant de 1 à 4 atomes de carbone ou alcoxy ayant de 1 à 4 atomes de carbone.

2. Dérivés d'acide amino-4 butanoïque selon la Revendication 1, de formule I dans laquelle $R_2$ représente un radical phényle.

3. Dérivés d'acide amino-4 butanoïque selon la Revendication 1, de formule I dans laquelle $R_2$ représente un radical hydroxy-4 phényle.

4. Dérivés d'acide amino-4 butanoïque selon la Revendication 1, de formule I dans laquelle R représente un radical tertiobutoxycarbonyl.

5. Dérivés d'acide amino-4 butanoïque selon la Revendication 1, de formule I dans laquelle $R_1$ représente un groupe méthyle.

6. (3S,4S)-hydroxy-3 N-BOC-amino-4 phényl-4 butanoate de méthyle.

7. (3R,4S)-hydroxy-3 N-BOC-amino-4 phényl-4 butanoate de méthyle.

8. Procédé de préparation de dérivés d'acide amino-4 butanoïque selon la Revendication 1, caractérisé en ce que :

a) lorsque $R_1$ représente un groupement labile, on hydrogène sous pression un dérivé (4S)-oxo-3 amino-4 butanoïque protégé de formule générale :

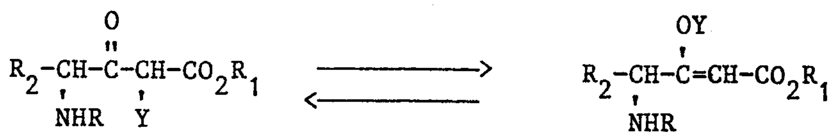

dans laquelle R et $R_2$ ont la même signification que dans la Revendication 1, le groupement hydroxy éventuellement présent étant protégé, $R_1$ représente un groupement labile et Y représente l'hydrogène ou un atome de métal alcalin, et ce, en présence d'un catalyseur à température ambiante et dans un solvant approprié, pour obtenir un mélange de diastéréoisomères,

b) lorsque $R_1$ représente un atome de métal alcalin, on saponifie au moyen d'un hydroxyde de métal alcalin un ester d'acide (3S,4S ; 3R, 4S)-hydroxy-3 amino-4 butanoïque préparé au paragraphe a) précédent et ce, dans un solvant approprié et à température ambiante pour obtenir les composés désirés sous forme de mélange de diastéréosiomères,

c) lorsque $R_1$ représente hydrogène, on acidifie à température ambiante au moyen d'un acide fort un sel d'acide (3S,4S ; 3R,4S)-hdyroxy-3 amino-4 butanoïque préparé au paragraphe b) précédent pour obtenir les composés désirés sous forme de mélange de diastéréoisomères,

ces diastéréoisomères pouvant, si on le désire, être séparés par chromatographie.

9. Procédé selon la Revendication 8, caractérisé en ce que le catalyseur utilisé en a) est le nickel de Raney.

10. Procédé selon la Revendication 8, caractérisé en ce que l'hydrogénation en a) est effectuée sous une pression d'environ 7 bars.

11. Procédé selon la Revendication 8, caractérisé en ce que le solvant utilisé en a) est le méthanol.

12. Procédé selon la Revendication 8, caractérisé en ce que le solvant utilisé en b) est le dioxanne aqueux.

13. Procédé selon la Revendication 8, caractérisé en ce que l'acide fort utilisé en c) est l'acide chlorhydrique.

14. Utilisation des dérivés d'acide (4S)-amino-4 butanoïque selon l'une quelconque des Revendications 1 à 7 pour la préparation de peptides dérivés d'analogues de statine.